# EUROPEAN PATENT SPECIFICATION

(11) **EP 3 641 739 B1**
(45) Date of publication and mention of the grant of the patent: **09.12.2026**
(21) Application number: 18733569.0
(22) Date of filing: 20.06.2018
(51) Int. Cl.: A61K 31/546, A61K 39/395, A61P 35/00, A61K 45/06

(54) **INHIBITOR OF SUV39H1 HISTONE METHYLTRANSFERASE FOR USE IN CANCER COMBINATION THERAPY**
INHIBITOR DER SUV39H1-HISTON-MEHTYLTRANSFERASE ZUR VERWENDUNG IN DER KREBSKOMBINATIONSTHERAPIE
INHIBITEUR DE MEHTYLTRANSFERASEHISTONE HISTONE SUV39H1 DESTINÉ À ÊTRE UTILISÉ DANS UNE THÉRAPIE COMBINÉE ANTI-CANCÉREUSE

(30) Priority: 20.06.2017 EP 17305755
(43) Date of publication of application: 29.04.2020
(73) Proprietor: Institut Curie, 75005 Paris (FR); Institut National de la Santé et de la Recherche Médicale (INSERM), 75013 Paris (FR)
(72) Inventor: AMIGORENA, Sebastian, 75013 Paris (FR); PIAGGIO, Eliane, 75020 Paris (FR); NIBORSKI, Leticia, 75018 Paris (FR)
(74) Representative: Plasseraud IP
(86) International application number: PCT/EP2018/066383
(87) International publication number: WO 2018/234367

(56) References cited:
- WO-A1-2016/200690
- DATABASE EMBASE [online] ELSEVIER SCIENCE PUBLISHERS, AMSTERDAM, NL; 1 July 2018 (2018-07-01), LU C ET AL: "The SUV39H1-H3K9me3 axis mediates colon carcinoma cell intrinsic apoptosis and immune evasion", Database accession no. EMB-623508795
- LU C ET AL: "The SUV39H1-H3K9me3 axis mediates colon carcinoma cell intrinsic apoptosis and immune evasion", CANCER RESEARCH 20180701 AMERICAN ASSOCIATION FOR CANCER RESEARCH INC. NLD, vol. 78, no. 13, Supplement 1, 1 July 2018 (2018-07-01), ISSN: 1538-7445
- DATABASE BIOSIS [online] BIOSCIENCES INFORMATION SERVICE, PHILADELPHIA, PA, US; June 2017 (2017-06-01), LU CHUNWAN ET AL: "The MLL1-H3K4me3 Axis-Mediated PD-L1 Expression and Pancreatic Cancer Immune Evasion", Database accession no. PREV201700581887
- JNCI-JOURNAL OF THE NATIONAL CANCER INSTITUTE, vol. 109, no. 6, June 2017 (2017-06-01), pages Article No.: djw283, ISSN: 0027-8874(print), DOI: 10.1093/JNCI/DJW283
- LU CHUNWAN ET AL: "The MLL1-H3K4me3 Axis-Mediated PD-L1 Expression and Pancreatic Cancer Immune Evasion", JNCI-JOURNAL OF THE NATIONAL CANCER INSTITUTE,, vol. 109, no. 6, 29 January 2017 (2017-01-29), XP002776050, DOI: 10.1093/JNCI/DJW283
- CHUNWAN LU ET AL: "The MLL1-H3K4me3 Axis-Mediated PD-L1 Expression and Pancreatic Cancer Immune Evasion", JOURNAL OF THE NATIONAL CANCER INSTITUTE, vol. 109, no. 6, DJW283, 28 January 2017 (2017-01-28), GB, pages 1-12 - S1-S16, XP055429970, ISSN: 0027-8874, DOI: 10.1093/jnci/djw283
- AMY V. PASCHALL ET AL: "H3K9 Trimethylation Silences Fas Expression To Confer Colon Carcinoma Immune Escape and 5-Fluorouracil Chemoresistance", THE JOURNAL OF IMMUNOLOGY, vol. 195, no. 4, 1 July 2015 (2015-07-01), US, XP055429086, ISSN: 0022-1767, DOI: 10.4049/jimmunol.1402243
- CHUNWAN LU ET AL: "Epigenetic regulation of PD-L1 expression and pancreatic cancer response to checkpoint immunotherapy", TRANSLATIONAL CANCER RESEARCH, vol. 6, no. S3, 1 May 2017 (2017-05-01), pages S652 - S654, XP055429144, ISSN: 2218-676X, DOI: 10.21037/tcr.2017.05.32
- H. ÜMIT KANISKAN ET AL: "Selective Inhibitors of Protein Methyltransferases", JOURNAL OF MEDICINAL CHEMISTRY, vol. 58, no. 4, 2 December 2014 (2014-12-02), pages 1596 - 1629, XP055429259, ISSN: 0022-2623, DOI: 10.1021/jm501234a
- ROBERT D. LEONE ET AL: "A2aR antagonists: Next generation checkpoint blockade for cancer immunotherapy", COMPUTATIONAL AND STRUCTURAL BIOTECHNOLOGY JOURNAL, vol. 13, 1 January 2015 (2015-01-01), Sweden, pages 265 - 272, XP055429269, ISSN: 2001-0370, DOI: 10.1016/j.csbj.2015.03.008
- H-J JUNG ET AL: "The anticancer effect of chaetocin is enhanced by inhibition of autophagy", CELL DEATH AND DISEASE, vol. 7, no. 2, 18 February 2016 (2016-02-18), pages e2098, XP055429274, DOI: 10.1038/cddis.2016.15
- DATABASE EMBASE [online] ELSEVIER SCIENCE PUBLISHERS, AMSTERDAM, NL; 1 July 2018 (2018-07-01), LU C ET AL: "The SUV39H1-H3K9me3 axis mediates colon carcinoma cell intrinsic apoptosis and immune evasion", XP002784552, Database accession no. EMB-623508795
- CANCER RESEARCH 20180701 AMERICAN ASSOCIATION FOR CANCER RESEARCH INC. NLD, vol. 78, no. 13, Supplement 1, 1 July 2018 (2018-07-01), ISSN: 1538-7445
- A. ZIPPELIUS ET AL: "Induced PD-L1 Expression Mediates Acquired Resistance to Agonistic Anti-CD40 Treatment", CANCER IMMUNOLOGY RESEARCH, vol. 3, no. 3, 26 January 2015 (2015-01-26), pages 236 - 244, XP055218169, ISSN: 2326-6066, DOI: 10.1158/2326-6066.CIR-14-0226
- ANONYMOUS: "Phase 1/2 Clinical Trial of Apexigen's APX005M in Combination with Pembrolizumab (Keytruda ) Opens for Metastatic Melanoma Patients", 21 March 2017 (2017-03-21), pages 1 - 2, XP055505702, Retrieved from the Internet <URL:https://www.apexigen.com/news/phase-1-2-clinical-trial-of-apexigens-apx005m-in-combination-with-pembrolizumab-keytruda-opens-for-metastatic-melanoma-patients/> [retrieved on 20180910]
- P ELLMARK ET AL: "Kick-starting the cancer-immunity cycle by targeting CD40", ONCOIMMUNOLOGY, vol. 4, no. 7, 16 March 2015 (2015-03-16), pages e1011484, XP055425223, DOI: 10.1080/2162402X.2015.1011484
- SIWEN HU-LIESKOVAN ET AL: "New Combination Strategies Using Programmed Cell Death 1/Programmed Cell Death Ligand 1 Checkpoint Inhibitors as a Backbone :", CANCER JOURNAL, vol. 23, no. 1, 1 February 2017 (2017-02-01), US, pages 10 - 22, XP055505708, ISSN: 1528-9117, DOI: 10.1097/PPO.0000000000000246
- QIAN BIAN ET AL: "[beta]-Globin cis-elements determine differential nuclear targeting through epigenetic modifications", THE JOURNAL OF CELL BIOLOGY : JCB, vol. 203, no. 5, 2 December 2013 (2013-12-02), US, pages 767-783 - S1-S9, XP055505726, ISSN: 0021-9525, DOI: 10.1083/jcb.201305027

## Description

### FIELD OF THE INVENTION

The present invention relates to an inhibitor of H3K9 histone methyl transferase SUV39H1 gene expression selected from anti-sense oligonucleotide constructs, siRNAs, shRNAs and ribozymes targeting SUV39H1 mRNA, for use in combination with at least one inhibitor of an inhibitory immune checkpoint molecule that is an anti-Programmed Death-Ligand 1 (PD-L1) antibody, in the treatment of cancer.

### BACKGROUND OF THE INVENTION

Immune checkpoints refer to a plethora of inhibitory and stimulatory pathways hardwired into the immune system that are crucial for maintaining self-tolerance and modulating the duration and amplitude of physiological immune responses in peripheral tissues, in order to minimize collateral tissue damage. Indeed, the balance between inhibitory and stimulatory signals determines the lymphocyte activation and consequently regulates the immune response (Pardoll DM, Nat Rev Cancer. 2012 Mar 22;12(4):252-64).

It is now clear that tumours co-opt certain immune-checkpoint pathways as a major mechanism of immune resistance, particularly against T cells that are specific for tumour antigens. Because many of the immune checkpoints are initiated by ligand-receptor interactions, they can be readily blocked by antibodies or modulated by recombinant forms of ligands or receptors. Thus agonists of co-stimulatory receptors or antagonists of inhibitory signals, both of which result in the amplification of antigen-specific T cell responses are the primary agent in current clinical testing.

In this context, cancer immunotherapy has been viewed as breakthrough in the field of cancer treatment, switching from targeting the tumor to targeting the immune system (Couzin-Frankel J., Science. 2013 Dec 20;342(6165):1432-3). The blockade of immune checkpoints with antibodies anti-CTLA-4, PD1 and PD-L1 has given impressive clinical results and manageable safety profiles.

However, only a small proportion of patients respond to these therapies, thus, there is a need to improve cancer immunotherapies by new approaches and/or by combining anti-checkpoint antibodies with other treatments. Moreover, anti-checkpoint antibodies can induce side effects, mainly autoimmunity, such that implementing combination therapies which may help lower the administered doses, and consequently the adverse events, remains of invaluable medical help.

Epigenetic factors have also been implicated in cancer, inflammatory and autoimmune diseases, and in the past few years have been recognized as promising targets for drug development. Inhibitors of DNA methytransferase (DNMT) or of histone deacetylase (HDAC) are currently approved for clinical use in the treatment of haematological malignancies. Inhibitors of the histone methyltransferase, EZH2, have also been proposed for the treatment of patients with relapsed or refractory B-cell lymphoma (Nature. 2012 Dec 6;492(7427):108-12). The use of inhibitors of DNMT or HDAC has also been recently proposed in combination with other cancer therapies such as immunotherapy (WO2015035112, Chiapinelli KB et al., Cell. 2015 Aug 27;162(5):974-86; Licht JD Cell. 2015 Aug 27;162(5):938-9.) However, the role of such epigenetic modulators in cancer immunology and immunotherapy remains unclear poorly understood. Indeed, the effects of demethylating agents are diverse, and identification of genes, whose reactivation predicts or mediates response, remains elusive. Typically, immune modulatory effects of treatment with 5-Azacytidine, a DNMT, are complex and dependent on the clinical setting and type of patients (see Frösig TM and Hadrup SR, Mediators Inflamm. 2015; 2015: 871641). Chunwan Lu et al. (Journal of the National Cancer Institute, vol. 109, no. 6, DJW283, 28 Jan. 2017, pages 1-12-S1-S16) discloses the role of MLL1-H3K4Me3 axis in PD-L1 expression through the CD274 promoter and states that inhibition of MLL1 in combination with anti-PD-L1 or anti-PD-1 antibody immunotherapy is an efficient approach to suppress pancreatic tumor growth. It does not disclose the use of a selective SUV39H1 inhibitor in combination with a an inhibitory immune checkpoint molecule that is an anti-Programmed Death-Ligand 1 (PD-L1) antibody, in the treatment of cancer.

Thus there remains a need for implementing combination therapies that may improve efficacy of cancer immunotherapies with limiting adverse side effects.

### SUMMARY OF THE INVENTION

The present inventors have demonstrated for the first time that the anti-tumor effect of an immune checkpoint modulator is greatly enhanced in the absence of SUV39H1. In particular, they show that surprisingly, while anti-PD1 treatment, or suppression of SUV39H1 have only moderate anti-tumor effects separately, their combination leads to a massive and sustained tumor growth inhibition.

Furthermore, the relatively mild phenotype of *Suv39h1* knock-out mice suggests that blockade of this pathway would result in less collateral toxicity than other epigenetic treatments, such as inhibitors of DNA methyltransferases or inhibitors of EZH2.

Thus the present invention relates to an inhibitor of H3K9 histone methyl transferase SUV39H1 selected from anti-sense oligonucleotide constructs, siRNAs, shRNAs and ribozymes targeting SUV39H1 mRNA, for use in combination with at least one modulator of an immune checkpoint molecule that is anti-Programmed Death-Ligand 1 (PD-L1) antibody, in the treatment of cancer.

### DEFINITIONS:

Treatment", or "treating" as used herein, is defined as the application or administration of a therapeutic agent or combination of therapeutic agents (e.g., an inhibitor of SUV39H1 and/or an immune checkpoint modulator) to a patient, or application or administration of said therapeutic agents to an isolated tissue or cell line from a patient, who has a cancer with the purpose to cure, heal, alleviate, relieve, alter, remedy, ameliorate, improve or affect the cancer, or any symptom of the cancer. In particular, the terms "treat' or treatment" refers to reducing or alleviating at least one adverse clinical symptom associated with cancer, e.g., pain, swelling, low blood count etc.

In another embodiment, the term "treat' or treatment" refers to slowing or reversing the progression neoplastic uncontrolled cell multiplication, i.e. shrinking existing tumors and/or halting tumor growth.

The term "treat' or treatment" also refers to inducing apoptosis in cancer or tumor cells in the subject.

The term "treatment" or "treating" is also used herein in the context of administering the therapeutic agents prophylactically.

The term "effective dose" or "effective dosage" is defined as an amount sufficient to achieve, or at least partially achieve, the desired effect. The term "therapeutically effective dose" is defined as an amount sufficient to cure or at least partially arrest the disease and its complications in a patient already suffering from the disease. The term "patient" includes human and other mammalian subjects that receive either prophylactic or therapeutic treatment.

As used herein, the term "therapeutically effective regimen" refers to a regimen for dosing, timing, frequency, and duration of the administration of one or more therapies according to the invention (i.e., the inhibitor of SUV39H1 and the at least one immune checkpoint modulator), for the treatment and/or the management of cancer or a symptom thereof. In a specific embodiment, the regimen achieves one, two, three, or more of the following results: (1) a stabilization, reduction or elimination in the cancer cell population; (2) a stabilization or reduction in the growth of a tumor or neoplasm; (3) an impairment in the formation of a tumor; (4) eradication, removal, or control of primary, regional and/or metastatic cancer; (5) a reduction in mortality; (6) an increase in disease-free, relapse-free, progression-free, and/or overall survival, duration, or rate; (7) an increase in the response rate, the durability of response, or number of patients who respond or are in remission; (8) a decrease in hospitalization rate, (9) a decrease in hospitalization lengths, (10) the size of the tumor is maintained and does not increase or increases by less than 10%, preferably less than 5%, preferably less than 4%, preferably less than 2%, and (11) an increase in the number of patients in remission.

As used herein, the term "in combination", or "combined administration" in the context of the invention refers to the administration of an inhibitor of SUV39H1 and of at least one immune checkpoint modulator to a patient for cancer therapeutic benefit. The term "in combination" in the context of the administration can also refer to the prophylactic use of a SUV39H1 inhibitor when used with at least one immune checkpoint modulator.

The use of the term "in combination" does not restrict the order in which the therapies (*e.g*., SUV39H1 and the at least one immune checkpoint modulator) are administered to a subject. A therapy can be administered prior to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapy to a patient which had, has, or is susceptible to cancer. The therapies are administered to a patient in a sequence and within a time interval such that the therapies can act together. In a particular embodiment, the therapies are administered to a subject in a sequence and within a time interval such that they provide an increased benefit than if they were administered otherwise. Any additional therapy can be administered in any order with the other additional therapy.

These results of the present invention have established a basis for dual treatment of patients with an inhibitor of SUV39H1 and at least one immune checkpoint modulator such as an anti-PD-1 antibody. These two therapies need not be given concurrently, but can also be given sequentially, for example beginning with the SUV39H1 inhibitor and followed by immune checkpoint modulation. Accordingly, and as used herein, the expression "An inhibitor of H3K9 histone methyl transferase SUV39H1 for use in combination with at least one immune checkpoint modulator in the treatment of cancer" can be used interchangeably with the expression "At least one immune checkpoint modulator SUV39H1 for use in combination with an inhibitor of H3K9 histone methyl transferase SUV39H1 in the treatment of cancer".

The terms "synergy," "synergistic," or "synergistic effect" as used herein describe an effect that has a magnitude that is greater than the sum if the individual effects. In some embodiments of the present invention, the use of both a SUV39H1 inhibitor and an immune checkpoint modulator in concert provides a synergistic therapeutic effect on a neoplastic condition in a patient and/or on the growth of a cell. For example, if use of a SUV39H1 inhibitor produced a 10% reduction in tumor growth and use of an immune checkpoint modulator alone produced a 20% reduction in tumor growth, then the additive effect for reducing neoplastic or tumor growth would be 30% reduction. Hence, by comparison, a synergistic effect when using both the inhibitor of SUV39H1 and the immune checkpoint modulator would be reduction in tumor or neoplastic growth to any extent greater than 30% reduction.

As used herein, the term "antibody" refers to a protein that includes at least one immunoglobulin variable region, e.g., an amino acid sequence that provides an immunoglobulin variable domain or an immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (e.g., single chain antibodies, Fab fragments, F(ab')2 fragments, Fd fragments, Fv fragments, and dAb fragments) as well as complete antibodies, e.g., intact and/or full length immunoglobulins of types IgA, IgG (e.g., IgGI, IgG2, IgG3, IgG4), IgE, IgD, IgM (as well as subtypes thereof). The light chains of the immunoglobulin may be of kappa or lambda types. In one embodiment, the antibody is glycosylated. An antibody can be functional for antibody-dependent cytotoxicity and/or complement-mediated cytotoxicity, or may be non-functional for one or both of these activities.

### DETAILED DESCRIPTION OF THE INVENTION

### Inhibitors of SUV39H1

As used herein the term "SUV39H1" or "H3K9-histone methyltransferase SUV39H1" has its usual meaning in the art and refers to the histone methyltransferase "suppressor of variegation 3-9 homolog 1 (Drosophila)" that specifically trimethylates the Lys-9 residue of histone H3 using monomethylated H3-Lys-9 as substrate (see also Aagaard L, Laible G, Selenko P, Schmid M, Dorn R, Schotta G, Kuhfittig S, Wolf A, Lebersorger A, Singh PB, Reuter G, Jenuwein T (Jun 1999). "Functional mammalian homologues of the Drosophila PEV-modifier Su(var)3-9 encode centromere-associated proteins which complex with the heterochromatin component M3 1 " . EMBO J 1 8 (7): 1923-38.). Said histone methyltransferase is also known as MG44, KMT1A, SUV39H, histone- lysine N-methyltransferase SUV39H1, H3-K9-HMTase 1, OTTHUMP00000024298, Su(var)3-9 homolog 1, lysine N-methyltransferase 1A, histone H3-K9 methyltransferase 1, position-effect variegation 3-9 homolog, histone- lysine N-methyltransferase, or H3 lysine-9 specific 1. The human SUV39H1 methyltransferase is referenced O43463 in UNIPROT. The term SUV39H1 also encompasses all orthologs of SUV39H1 such as SU(VAR)3-9. SUV39H1 is a critical player for gene silencing however its pathway to gene silencing is completely distinct and independent from other typical histone methyl transferases such as EZH2. Indeed while EZH2-KO mice are not viable (O'Carroll D et al., Mol Cell Biol. 2001 Jul;21(13):4330-6.), Suv39h1 KO mice in contrast display only minor phenotypes. Accordingly, the roles of SUV39H1 and EZH2 in immune responses are also distinct. Conditional KOs for EZH2 in lymphocytes causes a profound defect in lymphocyte development. On the contrary, the immune system of Suv39h1 KO mice is not affected. Therefore, SUV39H1 was not a predictable relevant target for immune manipulations. In this context, the results of the present invention are highly unexpected.

The inhibiting activity of an inhibitor of SUV39H1 may be determined using various methods as described in Greiner D. Et al. Nat Chem Biol. 2005 Aug;I(3): 143-5 or Eskeland, R. et al. Biochemistry 43, 3740-3749 (2004).

Inhibitors of H3K9 histone methyl transferase SUV39H1 gene expression are selected from anti-sense oligonucleotide constructs, siRNAs, shRNAs and ribozymes targeting SUV39H1 mRNA.

Anti-sense oligonucleotides, including anti-sense RNA molecules and anti-sense DNA molecules, would act to directly block the translation of H3K9-histone methyltransferase SUV39H1 and thus preventing protein translation or increasing mRNA degradation, thus decreasing the level of H3K9-histone methyltransferase SUV39H1 and thus its activity in a cell. For example, antisense oligonucleotides of at least 15 bases and complementary to unique regions of the mRNA transcript sequence encoding H3K9-histone methyltransferase SUV39H1 can be synthesized, *e.g.,* by conventional phosphodiester techniques and administered by *e.g.,* intravenous injection or infusion. Methods for using antisense techniques for specifically inhibiting gene expression of genes whose sequence is known are well known in the art (see for example U.S. Pat. Nos. 6,566,135; 6,566, 131; 6,365,354; 6,410,323; 6,107,091; 6,046,321 ; and 5,981,732).

Small inhibitory RNAs (siRNAs) can also function as inhibitors of expression for use in the present invention. H3K9-histone methyltransferase SUV39H1 gene expression can be reduced by contacting a subject or cell with a small double stranded RNA (dsRNA), or a vector or construct causing the production of a small double stranded RNA, such that H3K9-histone methyltransferase SUV39H1 gene expression is specifically inhibited (*i.e*. RNA interference or RNAi). Methods for selecting an appropriate dsRNA or dsRNA-encoding vector are well known in the art for genes whose sequence is known (see for example Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836). All or part of the phosphodiester bonds of the siRNAs of the invention are advantageously protected. This protection is generally implemented via the chemical route using methods that are known in the art. The phosphodiester bonds can be protected, for example, by a thiol or amine functional group or by a phenyl group. The 5'- and/or 3'- ends of the siRNAs of the invention are also advantageously protected, for example, using the technique described above for protecting the phosphodiester bonds. The siRNA sequences advantageously comprise at least twelve contiguous dinucleotides or their derivatives.

As used herein, the term "siRNA derivatives" with respect to the present nucleic acid sequences refers to any nucleic acid having a percentage of identity of at least 90% with erythropoietin or fragment thereof, preferably of at least 95%, as an example of at least 98%, and more preferably of at least 98%.

As used herein, the expression "percentage of identity" between two nucleic acid sequences, means the percentage of identical nucleic acid, between the two sequences to be compared, obtained with the best alignment of said sequences, this percentage being purely statistical and the differences between these two sequences being randomly spread over the nucleic acid acids sequences. As used herein, "best alignment" or "optimal alignment", means the alignment for which the determined percentage of identity (see below) is the highest. Sequence comparison between two nucleic acids sequences is usually realized by comparing these sequences that have been previously aligned according to the best alignment; this comparison is realized on segments of comparison in order to identify and compare the local regions of similarity. The best sequences alignment to perform comparison can be realized, besides manually, by using the global homology algorithm developed by SMITH and WATERMAN (Ad. App. Math., vol.2, p:482, 1981), by using the local homology algorithm developed by NEDDLEMAN and WUNSCH (J. Mol. Biol, vol.48, p:443, 1970), by using the method of similarities developed by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C, Nucleic Acids Research, vol. 32, p: 1792, 2004). To get the best local alignment, one can preferably use BLAST software. The identity percentage between two sequences of nucleic acids is determined by comparing these two sequences optimally aligned, the nucleic acids sequences being able to comprise additions or deletions in respect to the reference sequence in order to get the optimal alignment between these two sequences. The percentage of identity is calculated by determining the number of identical positions between these two sequences, and dividing this number by the total number of compared positions, and by multiplying the result obtained by 100 to get the percentage of identity between these two sequences.

shRNAs (short hairpin RNA) can also function as inhibitors of expression for use in the present invention.

Ribozymes can also function as inhibitors of expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleo lytic cleavage. Engineered hairpin or hammerhead motif ribozyme molecules that specifically and efficiently catalyze endonucleolytic cleavage of H3K9-histone methyltransferase SUV39H1 mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between 15 and 20 ribonucleotides corresponding to the region of the target gene containing the cleavage site can be evaluated for predicted structural features, such as secondary structure, that can render the oligonucleotide sequence unsuitable.

Both antisense oligonucleotides and ribozymes useful as inhibitors of expression can be prepared by known methods. These include techniques for chemical synthesis such as, *e.g.,* by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-0-methyl rather than phosphodiesterase linkages within the oligonucleotide backbone.

Antisense oligonucleotides, siRNAs, shRNAs and ribozymes of the invention may be delivered in vivo alone or in association with a vector. In its broadest sense, a "vector" is any vehicle capable of facilitating the transfer of the antisense oligonucleotide, siRNA, shRNA or ribozyme nucleic acid to the cells and preferably cells expressing H3K9-histone methyltransferase SUV39H1. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the antisense oligonucleotide, siRNA, shRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rous sarcoma virus; adenovirus, adeno-associated virus; SV40-type viruses; polyoma viruses; Epstein-Barr viruses; papilloma viruses; herpes virus; vaccinia virus; polio virus; and R A virus such as a retrovirus. One can readily employ other vectors not named but known in the art.

Preferred viral vectors are based on non-cytopathic eukaryotic viruses in which nonessential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adenoviruses and adeno-associated (AAV) viruses, which are double-stranded DNA viruses that have already been approved for human use in gene therapy. Currently, 12 different AAV serotypes (AAV1 to 12) are known, each with different tissue tropisms (Wu, Z Mol Ther 2006; 14:316-27). Recombinant AAVs are derived from the dependent parvovirus AAV2 (Choi, VW J Virol 2005; 79:6801-07). The adeno-associated virus type 1 to 12 can be engineered to be replication deficient and is capable of infecting a wide range of cell types and species (Wu, Z Mol Ther 2006; 14:316-27). It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al, 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate delivery vehicles and micro encapsulation.

The antisense oligonucleotide, siRNA, shRNA or ribozyme nucleic acid sequence according to the invention is generally under the control of a heterologous regulatory region, e.g., a heterologous promoter. The promoter may be specific for Muller glial cells, microglia cells, endothelial cells, pericyte cells and astrocytes For example, a specific expression in Muller glial cells may be obtained through the promoter of the glutamine synthetase gene is suitable. The promoter can also be, as a matter of example, a viral promoter, such as CMV promoter or any synthetic promoters.

### Immune checkpoint modulators

As used herein the term "immune checkpoint protein" has its general meaning in the art and refers to a molecule that is expressed by T cells and/or by NK cells and that either turn up a signal (stimulatory checkpoint molecules) or turn down a signal (inhibitory checkpoint molecules). Most preferably according to the invention the immune checkpoint molecule is at least expressed by T cells.

Immune checkpoint molecules are recognized in the art to constitute immune checkpoint pathways similar to the CTLA-4 and PD-1 dependent pathways. Immune checkpoint molecules are notably described in Pardoll, 2012. Nature Rev Cancer 12:252-264; Mellman et al., 2011. Nature 480:480- 489; Chen L & Flies DB, Nat. Rev. Immunol. 2013 April; 13(4):227-242, and Kemal Catakovic, Eckhard Klieser et al., "T cell exhaustion: from pathophysiological basics to tumor immunotherapy" Cell Communication and Signaling 2017,15:1). Example of immune checkpoints molecules notably encompasses CD27, CD40, OX40, GITR, ICOS, TNFRSF25, 41BB, HVEM, CD28, TMIGD2, CD226, 2B4 (CD244) and ligand CD48, B7-H6 Brandt (NK ligand), LIGHT (CD258, TNFSF14), CD28H, A2AR, B7-H3, B7-H4, BTLA, CTLA-4, CD277, IDO, KIRs, PD-1s, LAG-3, TIM-3 TIGIT, VISTA, CD96, CD112R, CD160, CD244 (or 2B4) DCIR (C-type lectin surface receptor), ILT3, ILT4 (Immunoglobulin-like transcript), CD31 (PECAM-1) (Ig-like R family), CD39, CD73, CD94/NKG2, GP49b (immunoglobulin superfamily), KLRG1, LAIR-1 (Leukocyte-associated immunoglobulin-like receptor 1) CD305 , PD-L1 and PD-L2 and SIRPα

According to the invention, the inhibitor of H3K9 histone methyl transferase SUV39H1 gene expression is for use in combination with at least one inhibitor of an inhibitory immune checkpoint molecule that is an anti-Programmed Death-Ligand 1 (PD-L1) antibody.

According to the present invention, more than one modulator of an immune checkpoint protein can be used in combination with the inhibitor of SUV39H1 according to the present invention. For example, at least one immune checkpoint molecule that is an anti-PD-L1 antibody can be used in combination with at least one stimulatory immune checkpoint agonist. Co-stimulatory and co-inhibitory immune checkpoint molecules are notably described in the review of Chen L & Flies B (Nat rev Immuno., 2013 mentioned above).

Non-limitative examples of inhibitory checkpoint molecules include A2AR, B7-H3, B7-H4, BTLA, CTLA-4, CD277, IDO, KIRs, PD-1, LAG-3, TIM-3 TIGIT, VISTA, CD96, CD112R, CD160, DCIR (C-type lectin surface receptor), ILT3, ILT4 (Immunoglobulin-like transcript), CD31 (PECAM-1) (Ig-like R family), CD39, CD73, CD94/NKG2, GP49b (immunoglobulin superfamily), KLRG1, LAIR-1 (Leukocyte-associated immunoglobulin-like receptor 1), CD305 , PD-L1 and PD-L2.

The Adenosine A2a receptor (A2aR), the ligand of which is adenosine, is regarded as an important checkpoint in cancer therapy because adenosine in the immune microenvironment, leading to the activation of the A2a receptor, is negative immune feedback loop and the tumor microenvironment has relatively high concentrations of adenosine. A2aR can be inhibited by antibodies that block adenosine binding or by adenosine analogues some of which are fairly specific for A2aR. These drugs have been used in clinical trials for Parkinson's disease.

The B7 family is an important family of membrane-bound ligand that binds co-stimulatory and inhibitory receptors. All of the B7 family members and their known ligands belong to the immunoglobulin superfamily. Many receptors have not been yet identified. B7-H3, also called CD276, was originally understood to be a co-stimulatory molecule but is now regarded as co-inhibitory. B7-H4, also called VTCN1, is expressed by tumor cells and tumor-associated macrophages and plays a role in tumour escape.

CD160 is a glycosylphosphatidylinositol (GPI)-anchored protein member of the Ig superfamily with a restricted expression profile that is limited to CD56dim CD16+ NK cells, NKT-cells, γδ T-cells, cytotoxic CD8+ T-cells lacking the expression of CD28, a small fraction of CD4+ T cells and all intraepithelial lymphocytes. Binding of CD160 to both classical and non-classical MHC I enhances NK and CD8+ CTL functions. However, engagement of CD160 by the Herpes Virus Entry Mediator (HVEM / TNFRSF14) was shown to mediate inhibition of CD4+ T-cell proliferation and TCR-mediated signaling.

HVEM (Herpesvirus Entry Mediator) protein is a bimolecular switch that binds both co-stimulatory LT-α/LIGHT and co-inhibitory receptors BTLA/CD160. The ligation of coinhibitory receptors BTLA and/or CD160 on T cells with HVEM expressed on DC or Tregs transduces negative signals into T cells that are counterbalanced by costimulatory signals delivered after direct engagement of HVEM on T cells by LIGHT expressed on DC or more likely, on other activated T cells (T-T cell cooperation). The predominance of the interaction of HVEM with BTLA and CD160 over the HVEM/LIGHT pathway or vice versa might be the result of differences in ligand/receptor affinity and the differential expression pattern of these molecules on cell types at different stages of cell differentiation. LIGHT, BTLA, and CD160 have substantially different binding affinities and occupy spatially distinct sites upon interaction with the HVEM receptor, which enables HVEM to function as a molecular switch. The net effect of the LIGHT/HVEM and HVEM/BTLA/CD160 interaction, when these different receptors and ligands are simultaneously present, determines the outcome of the response (see M. L. del Rio. "HVEM/LIGHT/BTLA/CD160 cosignaling pathways as targets for immune regulation" Journal of Leukocyte Biology. 2010; 87).

B and T Lymphocyte Attenuator (BTLA), also called CD272, has also HVEM as its ligand. BTLA T cells are inhibited in the presence of its ligand, HVEM. Surface expression of BTLA is gradually downregulated during differentiation of human CD8+ T cells from the naive to effector cell phenotype, however tumor-specific human CD8+ T cells express high levels of BTLA (Kenneth M. Murphy et al. Balancing co-stimulation and inhibition with BTLA and HVEM. Nature Reviews Immunology 2006, 6, 671-681).

CTLA-4, Cytotoxic T-Lymphocyte-Associated protein 4 also called CD152, was the first immune checkpoint to be clinically targeted. It is expressed exclusively on T cells. It has been proposed that its expression on the surface of T cells dampens the activation of T cells by outcompeting CD28 in binding CD80 and CD86 as well as actively delivering inhibitory signals to the T cells. Expression of CTLA-4 on Treg cells serves to control T cell proliferation.

Ig-like transcript-3 and -4 (ILT3 and ILT4) are inhibitory receptors both expressed by monocytes, macrophages, and DCs. The corresponding ILT3 ligand is not yet known, but since ILT3 can directly suppress T lymphocyte function, it is likely to be expressed on T cells. In several cancers, ILT3 has been found to mediate the immune escape mechanism by impairing T cell responses. Furthermore, ILT4-expressing DCs block efficient CTL differentiation, a mechanism that is used by tumors, which upregulate ILT4 to evade the immune system (Vasaturo A et al., Front Immunol. 2013; 4: 417).

Platelet endotheial cell adhesion molecule-1 (PECAM-1), also known as CD31, is a type I transmembrane glycoprotein member of the immunoglobulin (Ig) gene superfamily which contains six extracellular Ig domains and two cytoplasmic immunoreceptor tyrosine-based inhibitory motifs (ITIMs). PECAM-1 is restricted to endothelial cells and cells of the hematopoietic system (see Newman DK, Fu G, Adams T, et al. The adhesion molecule PECAM-1 enhances the TGFβ-mediated inhibition of T cell function. Science signaling. 2016;9(418):ra27).

LAIR-1 is expressed in very high and relatively homogenous levels in naive T cells but in lower and more heterogeneous levels in memory T cells. LAIR-1 consist of a type I transmembrane glycoprotein of 287 amino acids with a single extracellular C2-type Iglike domain and a cytoplasmic domain with two ITIM motifs. LAIR-1 can inhibit TCR mediated signals possibly through the recruitment of C-terminal Csk, one or more of the phosphatases SHIP, SHP-1 or SHP-2, and to a certain extent on signalling through p38 MAP kinase and ERK signaling (Thaventhiran T et al. (2012) J Clin Cell Immunol S12:004).

IDO1, Indoleamine 2,3-dioxygenase 1, is a tryptophan catabolic enzyme. A related immune-inhibitory enzymes. Another important molecule is TDO, tryptophan 2,3-dioxygenase. IDO1 is known to suppress T and NK cells, generate and activate Tregs and myeloid-derived suppressor cells, and promote tumour angiogenesis.

KIR, Killer-cell Immunoglobulin-like Receptor, are a braid category of inhibitory receptors that can be divided into two classes based on structure: killer cell immunoglobulin-like receptors (KIRs) and C-type lectin receptors which are type II transmembrane receptors. There receptors where originally described as regulators of the killing acitivity of NK cells although many are expressed on T cells and APCs. Many if the KIRs are soecufuc for subsets MHC class I molecules and possess allele-specificity.

LAG3, Lymphocyte Activation Gene-3 has, as its ligand, MHC class II molecules, which are upregulated on some epithelial cancers but are also expressed on tumour-infiltrating macrophages and dendritic cells. This immune checkpoint works to suppress an immune response by action to T_{reg} cells as well as direct effects on CD8+ T cells.

PD-1, Programmed Death 1 (PD-1) receptor, has two ligands, PD-L1 and PD-L2. This checkpoint is the target of Merck & Co.'s melanoma drug Keytruda, which gained FDA approval in September 2014. An advantage of targeting PD-1 is that it can restore immune function in the tumor microenvironment.

TIM-3 short for T-cell Immunoglobulin domain and Mucin domain 3 (also named B7H5), and the ligand of which is galacting 9, is expressed on activated human CD4+ T cells and regulates Th1 and Th17 cytokines. TIM-3 acts as a negative regulator of Th1/Tc1 function by triggering cell death upon interaction with its ligand, galectin-9.

VISTA (short for V-domain Ig suppressor of T cell activation) VISTA, also known as c10orf54, PD-1H, DD1α, Gi24, Dies1, and SISP1] is a member of the B7 family of NCRs and represents a new target for immunotherapy. Murine VISTA is a type I transmembrane protein with a single IgV domain with sequence homology to its B7 relatives with conserved segments thought to be critical for the IgV stability. VISTA is expressed on naïve T cells whereas PD-1 and CTLA-4 are not, which may suggest that VISTA functions to restrain T cell activity at an even earlier stage in T cell priming. VISTA is expressed on both T cells and APCs with very high expression on myeloid cells. VISTA is hematopoietically restricted and in multiple cancer models, VISTA was only detected on tumor infiltrating leukocytes and not on tumor cells. This unique surface expression pattern suggests that VISTA may function to restrict T cell immunity at different stages. VISTA has been demonstrated to exert both ligand and receptor functions. First, VISTA can function as a ligand to negatively regulate T cell activation. Second, VISTA has been demonstrated to function as a receptor on T cells which negatively regulates their activity. VISTA^{-/-} CD4⁺ T cells respond more vigorously than wild type (WT) CD4⁺ T cells to both polyclonal and antigen specific stimulation leading to increased proliferation and production of IFNγ, TNFα, and IL-17A. Anti-VISTA monotherapy reduced tumor growth in multiple pre-clinical models, B16OVA melanoma, B16-BL6 melanoma, MB49 bladder carcinoma, and PTEN/BRAF inducible melanoma (see Deng J, Le Mercier I, Kuta A, Noelle RJ. "A New VISTA on combination therapy for negative checkpoint regulator blockade. J Immunother Cancer. 2016 Dec 20;4:86. doi: 10.1186/s40425-016-0190-5. eCollection 2016. Review; see also Kathleen M. Mahoney et al., "Combination cancer immunotherapy and new immunomodulatory targets". Nature Reviews Drug Discovery 2015; 14:561-584).

CD96, CD226 (DNAM-1) and TIGIT belong to an emerging family of receptors that interact with nectin and nectin-like proteins. CD226 activates natural killer (NK) cell-mediated cytotoxicity, whereas TIGIT reportedly counterbalances CD226.

CD96 competes with CD226 for CD155 binding and limites NK cell function by direct inhibition (Christopher J Chan et al., "The receptors CD96 and CD226 oppose each other in the regulation of natural killer cell functions", Nature Immunology 2014 15, 431-438).

TIGIT (also called T cell immunoreceptor with Ig and ITIM domains, or VSTM3) TIGIT / VSTM3 is expressed normally by activated T cells, regulatory T (T_{reg}) cells, and natural killer (NK) cells. The poliovirus receptor (CD155 / PVR) and Nectin-2 (CD112) as well as CD 113 have been identified as relevant ligands. TIGIT / VSTM3 competes with the molecules CD226 and CD96 for binding to CD155 / PVR and CD112, respectively, but among all respective receptor-ligand combinations, TIGIT / VSTM3 exhibits the strongest affinity for CD155 / PVR. TIGIT inhibits T cell activation in vivo (see Karsten Mahnke et al. TIGIT-CD155 Interactions in Melanoma: A Novel Co-Inhibitory Pathway with Potential for Clinical Intervention. Journal of Investigative Dermatology. 2016; 136: 9-11).

CD112R (PVRIG), the ligand of which is PVRL2, is a member of poliovirus receptor-like proteins which is preferentially expressed on T cells and inhibits T cell receptor-mediated signals.

Non-limitative examples of stimulatory checkpoint molecules include CD27, CD40L, OX40, GITR, ICOS, TNFRSF25, 41BB, HVEM, CD28, TMIGD2, and CD226, 2B4 (CD244) and its ligand CD48, B7-H6 Brandt (NK ligand), CD28H and LIGHT (CD258, TNFSF14).

CD27, CD40L, OX40, GITR, ICOS, HVEM, 2B4 (CD244) and its ligand CD48, B7-H6 Brandt (NK ligand), LIGHT (CD258, TNFSF14), CD28H and TNFSF25 are stimulatory checkpoint molecules, which are members of the tumor necrosis factor (TNF) receptor superfamily (TNFSF). TNFRSF proteins play an important role in B and T cell development, survival, and antitumor immune response. In addition, some TNFRSFs are involved in the deactivation of T_{reg} cells. Therefore, TNFRSF agonists activate tumor immunity, and their combination with immune checkpoint therapy is promising. Several antibodies that act as TNFRSF agonist have been evaluated in clinical trials (Shiro Kimbara and Shunsuke Kondo, "Immune checkpoint and inflammation as therapeutic targets in pancreatic carcinoma", World J Gastroenterol. 2016 Sep 7; 22(33): 7440-7452, see also for review Watts TH. TNF/TNFR family members in costimulation of T cell responses. Annu Rev Immunol. 2005; 23:23-68.).

CD27 supports antigen-specific expansion of naïve T cells and is vital for the generation of T cell memory. CD27 is also a memory marker of B cells. CD27's activity is governed by the transient availability of its ligand, CD70, on lymphocytes and dendritic cells. CD27 costimulation is known to suppresses Th17 effector cell function

The CD40:CD40L pathway is a co-stimulatory pathway that affects both humoral and cell-mediated immunity.CD40L (also known as CD154), is primarily expressed on T-helper cells shortly after activation. The receptor 2B4 (CD244) belongs to the signaling lymphocyte activation molecule (SLAM) subfamily within the immunoglobulin superfamily (IgSV). All members of this family contain two or more immunoreceptor tyrosine-based switch motifs (ITSMs) in their cytoplasmatic tail including the receptors CD229, CS1, NTB-A and CD84 [92]. 2B4 is expressed by NK cells, γδ T cells basophils and monocytes, upon activation on CD8+ T cells and binds with high affinity to CD48 on lymphoid and myeloid cells (Kemal Catakovic et al., Cell Communication and Signaling201715:1).

TNFSF14 / LIGHT / CD258 exhibits inducible expression, and competes with herpes simplex virus (HSV) glycoprotein D for herpesvirus entry mediator (HVEM / TNFRSF14), a receptor expressed by T lymphocytes, is a recently identified member of the human and mouse TNF superfamily. TNFSF14 / LIGHT / CD258 is a 29-kD type II transmembrane protein produced by activated T cells, as well as monocytes and granulocytes, and immature DCs. In vitro, HVEM/LIGHT immune checkpoint pathway induces potent CD28-independent costimulatory activity, leading to NF-κB activation, production of IFN-γ and other cytokines, and T cell proliferation in response to allogeneic DCs. In vivo blockade studies show HVEM/LIGHT immune checkpoint pathway is involved in promotion of cytolytic T cell responses to tumors and the development of GVHD, and transgenic overexpression of TNFSF14 / LIGHT / CD258 within T cells leads to T cell expansion and causes various severe autoimmune diseases (Qunrui Ye et al. J Exp Med. 2002 Mar 18; 195(6): 795-800).

CD28H is constitutively expressed on all naive T cells. B7 homologue 5 (B7-H5), was identified as a specific ligand for CD28H. B7-H5 is constitutively found in macrophages and could be induced on dendritic cells. The B7-H5/CD28H interaction selectively costimulates human T-cell growth and cytokine production via an AKT-dependent signalling cascade (Zhu Y et al., Nat Commun. 2013; 4:204).

OX40, also called CD134, has OX40L, or CD252, as its ligand. Like CD27, OX40 promotes the expansion of effector and memory T cells, however it is also noted for its ability to suppress the differentiation and activity of T-regulatory cells, and also for its regulation of cytokine production. OX40's value as a drug target primarily lies it the fact that, being transiently expressed after T-cell receptor engagement, it is only upregulated on the most recently antigen-activated T cells within inflammatory lesions. Anti-OX40 monoclonal antibodies have been shown to have clinical utility in advanced cancer (Weinberg AD, Morris NP, Kovacsovics-Bankowski M, Urba WJ, Curti BD (November 1, 2011). "Science gone translational: the OX40 agonist story". Immunol Rev. 244 (1): 218-31).

GITR, short for Glucocorticoid-Induced TNFR family Related gene, prompts T cell expansion, including Treg expansion. The ligand for GITR (GITRL) is mainly expressed on antigen presenting cells. Antibodies to GITR have been shown to promote an anti-tumor response through loss of T_{reg} lineage stability (see Nocentini G, Ronchetti S, Cuzzocrea S, Riccardi C (May 1, 2007). "GITR/GITRL: more than an effector T cell co-stimulatory system". Eur J Immunol. 37 (5): 1165-9).

ICOS, short for Inducible T-cell costimulator, and also called CD278, is expressed on activated T cells. Its ligand is ICOSL, expressed mainly on B cells and dendritic cells. The molecule seems to be important in T cell effector function (Burmeister Y, Lischke T, Dahler AC, Mages HW, Lam KP, Coyle AJ, Kroczek RA, Hutloff A (January 15, 2008). "ICOS controls the pool size of effector-memory and regulatory T cells". J Immunol. 180 (2): 774-782).

Another stimulatory checkpoint molecules, which belongs to the B7-CD28 superfamily, are notably CD28 itself and TGMID2.

CD28 is constitutively expressed on almost all human CD4+ T cells and on around half of all CD8 T cells. Binding with its two ligands (CD80 and CD86, expressed on dendritic cells) prompts T cell expansion.

TMIGD2 (also called CD28 homolog), modulates T cell functions through interaction with its ligand HHLA2; a newly identified B7 family member. TMIGD2 protein is constitutively expressed on all naïve T cells and the majority of natural killer (NK) cells, but not on T regulatory cells or B cells (see Yanping Xiao and Gordon J. Freeman, "A new B7:CD28 family checkpoint target for cancer immunotherapy: HHLA2", Clin Cancer Res. 2015 May 15; 21(10): 2201-2203).

CD137 ligand (CD137L; also known as 4-1BBL and TNFSF9) is mainly expressed on professional antigen-presenting cells (APCs) such as dendritic cells, monocytes/macrophages, and B cells, and its expression is upregulated during activation of these cells. However, its expression has been documented on a variety of hematopoietic cells and nonhematopoietic cells. Generally, 4-1BBL / CD137L is constitutively expressed on many types of cells but its expression levels are low except for a few types of cells. Interestingly, 4-1BBL / CD137L is coexpressed with CD137 (also known as 4-1BB and TNFRSF9) on various types of cells, but expression of CD137 / 4-1BB potently downregulates that of 4-1BBL / CD137L by cis-interactions between the two molecules resulting in endocytosis of 4-1BBL / CD137L (see Byungsuk Kwon et al. Is CD137 Ligand (CD137L) "Signaling a Fine Tuner of Immune Responses?" Immune Netw. 2015 Jun;15(3):121-124).

Finally other immune checkpoint molecules also include CD244 (or 2B4) and SIRPα.

2B4 / CD244 is a member of the signaling lymphocyte activation molecule (SLAM)-related receptor family and is also known as SLAMF4 and CD244. All members of the SLAM family share a similar structure, including an extracellular domain, a transmembrane region, and a tyrosine rich cytoplasmic region. 2B4 & CD48 Immune Checkpoint Pathway can lead to signaling through both receptors. CD48 / SLAMF2 signaling in B cells leads to homotypic adhesion, proliferation and/or differentiation, release of inflammatory effector molecules and isotype class switching. In addition, all of these processes are also elicited in T cells via CD48 / SLAMF2 ligation with the addition of promoting their activation and/or cytotoxicity. 2B4 signaling requires signaling lymphocyte activation molecule (SLAM)-associated protein (SAP) or EWS-activated transcript 2 (EAT-2; also called SH2D1B). In CD8 T cells and NK cells 2B4 / CD244 has been reported to exert both positive and negative regulation (see also Sebastian Stark. "2B4 (CD244), NTB-A and CRACC (CS1) stimulate cytotoxicity but no proliferation in human NK cells". Int. Immunol. 2006 18 (2): 241-247).

CD47 is a cell surface glycoprotein with a variety of functions including regulation of phagocytosis through binding to the macrophage and dendritic cell specific protein signal regulatory protein alpha (SIRP alpha). Binding of SIRP alpha to CD47, as SIRP alpha & CD47 immune checkpoint pathway, essentially sends a "don't eat me" message to macrophages by initiating signaling to inhibit phagocytosis. Increased expression of CD47 is proposed to be a mechanism through which cancer cells evade immune detection and phagocytosis. Targeting of CD47 on cancer cells with an anti-CD47 blocking antibody can promote phagocytosis by macrophages in vitro. Further, treatment with an anti-CD47 blocking antibody synergized with rituximab treatment to promote phagocytosis in vitro and to eliminate cancer cells in an in vivo xenograft model of non-Hodgkin lymphoma. Further results demonstrate that CD47 expression increases in a variety of human solid tumor types and that blocking the SIRP alpha & CD47 immune checkpoint pathway with an anti-CD47 antibody can promote phagocytosis of solid tumor cells in vitro and reduce growth of solid tumors in vivo (see Martina Seiffert et al. "Signal-regulatory protein α (SIRPα) but not SIRPβ is involved in T-cell activation, binds to CD47 with high affinity, and is expressed on immature CD34+CD38-hematopoietic cells". 2001; Blood: 97 (9)).

As used herein, the expression "modulator of an immune checkpoint protein", or "checkpoint regulator cancer immunotherapy agent" (both expressions can be used interchangeably in the sense of the invention) has its general meaning in the art and refers to any compound inhibiting the function of an immune inhibitory checkpoint protein (inhibitory immune checkpoint inhibitors, or immune checkpoint inhibitors as previously described) or stimulating the function of a stimulatory checkpoint protein (stimulatory immune checkpoint agonist or immune checkpoint agonist used interchangeably). Inhibition includes reduction of function and full blockade.

The immune checkpoint modulators include peptides, antibodies, fusion proteins, nucleic acid molecules and small molecules. For certain immune checkpoint protein (i.e., immune pathway gene products), the use of either antagonists or agonists of such gene products is also contemplated, as are small molecule modulators of such gene products.

Preferred immune checkpoint inhibitors or agonists are antibodies, or fusions proteins that specifically recognize immune checkpoint proteins or their ligands, as described previously.

A fusion protein for use as immune checkpoint modulator can be made by fusion of a checkpoint molecule as described above with the crystallizable fragment (Fc) region of an immunoglobulin. Preferably antibodies are monoclonal antibodies.

A number of immune checkpoint inhibitors and agonists are known in the art and in analogy of these known immune checkpoint protein modulators, alternative immune checkpoint modulators may be developed in the (near) future and be used in combination with an inhibitor of SUV39H1 according to the invention.

An immune checkpoint modulator according to the invention results in an activation of the immune system and in particular leads to an amplification of antigen-specific T cell response. In particular, the immune checkpoint modulator of the present invention is administered for enhancing the proliferation, migration, persistence and/or cytoxic activity of CD8+ T cells in the subject and in particular the tumor-infiltrating of CD8+ T cells of the subject. As used herein "CD8+ T cells" has its general meaning in the art and refers to a subset of T cells which express CD8 on their surface. They are MHC class I-restricted, and function as cytotoxic T cells. "CD8+ T cells" are also called CD8+ T cells are called cytotoxic T lymphocytes (CTL), T-killer cell, cytolytic T cells, CD8+ T cells or killer T cells. CD8 antigens are members of the immunoglobulin supergene family and are associative recognition elements in major histocompatibility complex class I-restricted interactions. The ability of the immune checkpoint modulator to enhance T CD8 cell killing activity may be determined by any assay well known in the art. Typically said assay is an in vitro assay wherein CD8+ T cells are brought into contact with target cells (e.g. target cells that are recognized and/or lysed by CD8+ T cells).

For example, an immune checkpoint modulator can be selected for the ability to increase specific lysis by CD8+ T cells by more than 20%, preferably with at least 30%, at least 40%, at least 50%, or more of the specific lysis obtained at the same effector: target cell ratio with CD8+ T cells or CD8 T cell lines that are contacted by the immune checkpoint inhibitor of the present invention, Examples of protocols for classical cytotoxicity assays are conventional.

For example, a checkpoint regulator cancer immunotherapy agent can be an agent which blocks (an antagonist of) an immunosuppressive receptor (*i.e*., an inhibitory immune checkpoint) expressed by activated T lymphocytes, such as cytotoxic T lymphocyte-associated protein 4 (CTLA4) and programmed cell death 1 (PDCD1, best known as PD-1), or by NK cells, like various members of the killer cell immunoglobulin-like receptor (KIR) family).

Thus,, a checkpoint blockade cancer immunotherapy agent may be selected from the group consisting of anti-CTLA4 antibodies, anti-PD1 antibodies, anti-PDL2 antibodies, anti-TIM-3 antibodies, anti-LAG3 antibodies, anti-IDO1 antibodies, anti-TIGIT antibodies, anti-B7H3 antibodies, anti-B7H4 antibodies, anti-BTLA antibodies, anti-B7H6 antibodies, anti-CD86 antibodies, anti-Gal9 antibodies, anti-HVEM antibodies, anti-CD28 antibodies, anti-A2aR antibodies, anti-CD80 antibodies, anti-KIR(s) antibodies, A2aR drugs (notably adenosine analogs), anti-DCIR (C-type lectin surface receptor) antibodies, anti-ILT3 antibodies, anti-ILT4 antibodies, anti-CD31 (PECAM-1) antibodies, anti-CD39 antibodies, anti-CD73 antibodies, anti-CD94/NKG2 antibodies, anti-GP49b antibodies, anti-KLRG1 antibodies, anti-LAIR-1 antibodies, anti-CD305 antibodies, and their combinations. In certain embodiments, the checkpoint blockade cancer immunotherapy agent is an anti-PD- 1.

Examples of anti-CTLA-4 antibodies are described in US Patent Nos: 5,811,097; 5,811,097; 5,855,887; 6,051,227; 6,207,157; 6,682,736; 6,984,720; and 7,605,238. One anti-CDLA-4 antibody is tremelimumab, (ticilimumab, CP-675,206). In some embodiments, the anti-CTLA-4 antibody is ipilimumab (also known as 10D1, MDX-D010) a fully human monoclonal IgG antibody that binds to CTLA-4.

Examples of PD-1 and PD-L1 antibodies are described in US Patent Nos. 7,488,802; 7,943,743; 8,008,449; 8,168,757; 8,217,149, and PCT Published Patent Application Nos: WO03042402, WO2008156712, WO2010089411, WO2010036959, WO2011066342, WO2011159877, WO2011082400, and WO2011161699. In some embodiments, the PD-1 blockers include anti-PD-L1 antibodies. In certain other embodiments the PD-1 blockers include anti-PD-1 antibodies and similar binding proteins such as nivolumab (MDX 1106, BMS 936558, ONO 4538), a fully human IgG4 antibody that binds to and blocks the activation of PD-1 by its ligands PD-LI and PD-L2; lambrolizumab (MK-3475 or SCH 900475), a humanized monoclonal IgG4 antibody against PD-1; CT-011 a humanized antibody that binds PD-1 ; AMP-224 is a fusion protein of B7-DC; an antibody Fc portion; BMS-936559 (MDX- 1105-01) for PD-L1 (B7-H1) blockade.

Other immune-checkpoint inhibitors include lymphocyte activation gene-3 (LAG-3) inhibitors, such as IMP321, a soluble Ig fusion protein (Brignone et al., 2007, J. Immunol. 179:4202-4211).

Other immune-checkpoint inhibitors include B7 inhibitors, such as B7-H3 and B7-H4 inhibitors, notably, the anti-B7-H3 antibody MGA271 (Loo et al., 2012, Clin. Cancer Res. July 15 (18) 3834).

Also included are TIM3 (T-cell immunoglobulin domain and mucin domain 3) inhibitors (Fourcade et al., 2010, J. Exp. Med. 207:2175-86 and Sakuishi et al., 2010, J. Exp. Med. 207:2187-94). As used herein, the term "TIM-3" has its general meaning in the art and refers to T cell immunoglobulin and mucin domain-containing molecule 3. Accordingly, the term "TIM-3 inhibitor" as used herein refers to a compound, substance or composition that can inhibit the function of TIM-3. For example, the inhibitor can inhibit the expression or activity of TIM-3, modulate or block the TIM-3 signaling pathway and/or block the binding of TIM-3 to galectin-9, its natural ligand. Antibodies having specificity for TIM-3 are well known in the art and typically those described in WO2011155607, WO2013006490 and WO2010117057.

In some embodiments, the immune checkpoint inhibitor is an Indoleamine 2,3-dioxygenase (IDO) inhibitor, preferably an IDO1 inhibitor. Examples of IDO inhibitors are described in WO 2014150677. Examples of IDO inhibitors include without limitation 1-methyl-tryptophan (IMT), β- (3-benzofuranyl)-alanine, β-(3-benzo(b)thienyl)-alanine), 6-nitro-tryptophan, 6- fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'-diindolylmethane, epigallocatechin gallate, 5-Br-4-Cl-indoxyl 1,3-diacetate, 9-vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. Preferably the IDO inhibitor is selected from 1-methyl-tryptophan, β-(3- benzofuranyl)-alanine, 6-nitro-L-tryptophan, 3-Amino-naphtoic acid and β-[3- benzo(b)thienyl] -alanine or a derivative or prodrug thereof.

In some embodiments, the immune checkpoint inhibitor is an anti-TIGIT (T cell immunoglobin and ITIM domain) antibody.

In some embodiments, the immune checkpoint inhibitor is an anti-VISTA antibody, preferably a monoclonal antibody (Lines JL, Sempere LF, Wang L, et al. VISTA is an immune checkpoint molecule for human T cells. Cancer research. 2014; 74(7):1924-1932. doi:10.1158/0008-5472.CAN-13-1504).

In a preferred embodiment, the checkpoint modulator cancer immunotherapy agent is a combination of a CTLA4 blocking antibody, such as Ipilimumab, a PD-1 blocking antibody, such as Nivolumab or Pembrolizumab, and a PDL-1 blocking antibody.

A checkpoint modulator cancer immunotherapy agent can also be an agent, which activates a stimulatory immune checkpoint receptor expressed by activated T lymphocytes, or by NK cells, or an agent which mimics the principal ligands of these receptors, and results also in the amplification of antigen-specific T cell responses.

Thus, a checkpoint modulator cancer immunotherapy agent can typically be an agonistic antibody, notably a monoclonal agonistic antibody to a stimulatory immune checkpoint molecules as described above, for example selected from the group consisting of agonistic anti -4-1BB, -OX40, -GITR, -CD27, -ICOS, -CD40L, -TMIGD2, -CD226, - TNFSF25, -2B4 (CD244), - CD48, -B7-H6 Brandt (NK ligand), -CD28H -LIGHT (CD258, TNFSF14), and -CD28 antibodies.

A checkpoint agonist cancer immunotherapy agent can also be a fusion protein for example, a 4-1BB-Fc fusion protein, an Ox40-Fc fusion protein, a GITR-Fc fusion protein, a CD27-Fc fusion protein, an ICOS-Fc fusion protein, a CD40L-Fc fusion protein, a TMIGD2-Fc fusion protein, a CD226-Fc fusion protein, a TNFSF25-Fc fusion protein, a CD28-Fc fusion protein, a 2B4 (CD244) fusion protein, a CD48 fusion protein, a B7-H6 Brandt (NK ligand) fusion protein, a CD28H fusion protein and a LIGHT (CD258, TNFSF14) fusion protein.

Several of the 4-1BB agonists show great potential for application to human cancers. For example, BMS-666513, a fully humanized mAb against 4-1BB, has completed phase I and II trials for its anticancer properties in patients with melanoma, renal cell carcinoma, and ovarian cancer (Sznol M, Hodi FS, Margolin K, McDermott DF, Ernstoff MS, Kirkwood JM, et al. Phase I study of BMS-663513, a fully human anti-CD137 agonist monoclonal antibody, in patients (pts) with advanced cancer (CA). J Clin Oncol 26: 2008 (May 20 suppl; abstr 3007).

Seven OX40 agonists are now in development, 6 of which take the form of fully human monoclonal antibodies to address the mouse antibody issue. One OX40L-Fc fusion protein, MEDI6383, is also undergoing clinical evaluation; this links 2 OX40L molecules to part of the fragment crystallizable (Fc) region of immunoglobulin. In preclinical testing, the fusion protein appears to have stronger effects than OX40 antibodies, possibly because it may also activate dendritic cells and vascular endothelial cells in addition to T cells. Examples of Ox40 agonists include MEDI6469, MEDI6383, MEDI0652, PF-04515600, MOXP0916, GSK3174998, INCAGNO 1949.

Agonistic antibodies to GITR have been developed such as a humanized anti-human GITR mAb (TRX518 . Tolerx Inc. Agonistic antibodies to human glucocorticoid-induced tumor necrosis factor receptor as potential stimulators of T cell immunity for the treatment of cancer and viral infections. Expert Opin Ther Patents. 2007;17:567-575, see also Schaer DA, Murphy JT, Wolchok JD. Modulation of GITR for cancer immunotherapy. Curr Opin Immunol. 2012 Apr;24(2):217-24).

An example of an agonistic antibody to CD27, another member of the TNF family include the fully human 1F5 mAb that is now in Phase I clinical testing in B-cell malignancies, melanoma and renal cell carcinoma as CDX-1127 (varlilumab) (Analysis of the properties of the anti-CD27 monoclonal antibody (mAb) that is currently in clinical trials (Vitale LA, He L-Z, Thomas LJ et al. 2012 Development of a human monoclonal antibody for potential therapy of CD27-expressing lymphoma and leukemia. Clin. Cancer Res. 18(14), 3812-3821).

Initial clinical trials of agonistic CD40 mAb have shown highly promising results in the absence of disabling toxicity, in single-agent studies. To date, four CD40 mAb have been investigated in clinical trials: CP-870,893 (Pfizer and VLST), dacetuzumab (Seattle Genetics), Chi Lob 7/4 (University of Southampton), and lucatumumab (Novartis) (Vonderheide RH, Flaherty KT, Khalil M, Stumacher MS, Bajor DL, Hutnick NA, et al. Clinical activity and immune modulation in cancer patients treated with CP-870,893, a novel CD40 agonist monoclonal antibody. J Clin Oncol. 2007;25:876-83; Khubchandani S, Czuczman MS, Hernandez-Ilizaliturri FJ. Dacetuzumab, a humanized mAb against CD40 for the treatment of hematological malignancies. Curr Opin Investig Drugs. 2009;10:579-87; Johnson PW, Steven NM, Chowdhury F, Dobbyn J, Hall E, Ashton-Key M, et al. A Cancer Research UK phase I study evaluating safety, tolerability, and biological effects of chimeric anti-CD40 monoclonal antibody (MAb), Chi Lob 7/4. J Clin Oncol. 2010;28:2507; Bensinger W, Maziarz RT, Jagannath S, Spencer A, Durrant S, Becker PS, et al. A phase 1 study of lucatumumab, a fully human anti-CD40 antagonist monoclonal antibody administered intravenously to patients with relapsed or refractory multiple myeloma. Br J Haematol. 2012;159:58-66).

A checkpoint agonist cancer immunotherapy agent can also be an anti-ICOS agonist monoclonal antibody (Kutlu Elpek, Christopher Harvey, Ellen Duong, Tyler Simpson, Jenny Shu, Lindsey Shallberg, Matt Wallace, Sriram Sathy, Robert Mabry, Jennifer Michaelson, and Michael Briskin, Abstract A059: Efficacy of anti-ICOS agonist monoclonal antibodies in preclinical tumor models provides a rationale for clinical development as cancer immunotherapeutics; Abstracts: CRI-CIMT-EATI-AACR Inaugural International Cancer Immunotherapy Conference: Translating Science into Survival; September 16-19, 2015; New York, NY), or an anti-CD28 agonist antibody (for use notably in combination with anti-PD-1 immunotherapy, see T cell costimulatory receptor CD28 is a primary target for PD-1-mediated inhibition) see also Melero I, Hervas-Stubbs S, Glennie M, Pardoll DM, Chen L. Nat Rev Cancer. 2007 Feb;7(2):95-106, for review.

### Patients

Typically, the patient is a mammalian, preferably a human.

Typically said patient is suffering from a cancer, or is in remission or is at risk of a cancer. Targeting patients in remission (typically the patients who do not have any detectable tumor following for example surgical removal of the tumor) is of particular interest, as promoting tumor-free survival after cancer treatment represent one of the major concern in the field.

The cancer may be a solid cancer or a cancer affecting the blood (i.e., leukemia). Leukemia include for example acute myelogenous leukemia (AML), chronic myelogenous leukemia (CML), acute lymphocytic leukemia (ALL), and chronic lymphocytic leukemia, (including various lymphomas such as mantle cell lymphoma, non-Hodgkins lymphoma, adenoma, squamous cell carcinoma, laryngeal carcinoma, gallbladder and bile duct cancers, cancers of the retina such as retinoblastoma).

Solid cancers notably include cancers affecting one of the organs selected from the group consisting of colon, rectum, skin, endometrium, lung (including non-small cell lung carcinoma), uterus, bones (such as Osteosarcoma, Chondrosarcomas, Ewing's sarcoma, Fibrosarcomas, Giant cell tumors, Adamantinomas, and Chordomas), liver, kidney, esophagus, stomach, bladder, pancreas, cervix, brain (such as Meningiomas, Glioblastomas, Lower-Grade Astrocytomas, Oligodendrocytomas, Pituitary Tumors, Schwannomas, and Metastatic brain cancers), ovary, breast, head and neck region, testis, prostate and the thyroid gland.

### Dosage

Preferably the inhibitor of SUV39H1 and the immune checkpoint modulator are in an effective dose.

Typically the combined treatment regimen of the invention (i.e., the inhibitor of SUV39H1 and the at least one immune checkpoint modulator) is therapeutically effective. Currently available therapies and their dosages, routes of administration and recommended usage are known in the art and have been described in such literature as the Physician's Desk Reference (60th ed., 2006). Routes of administration include parenterally, intravenously, subcutaneously, intracranially, intrahepatically, intranodally, intraureterally, subureterally, subcutaneously, and intraperitoneally.

Dosage of one or more agents of the invention (e.g., SUV39H1 inhibitor and immune checkpoint modulator) can be determined by one of skill in the art and can also be adjusted by the individual physician in the event of any complication.

### Combination Therapies

In a specific embodiment, cycling therapy involves the administration of a first cancer therapeutic for a period of time, followed by the administration of a second cancer therapeutic for a period of time, optionally, followed by the administration of a third cancer therapeutic for a period of time and so forth, and repeating this sequential administration, i.e., the cycle in order to reduce the development of resistance to one of the cancer therapeutics, to avoid or reduce the side effects of one of the cancer therapeutics, and/or to improve the efficacy of the cancer therapeutics.

When two the two combined treatment according to the invention are administered to a patient concurrently, typically in a therapeutically effective regimen the term "concurrently" is not limited to the administration of the cancer therapeutics at exactly the same time, but rather, it is meant that they are administered to a subject in a sequence and within a time interval such that they can act together (e.g., synergistically to provide an increased benefit than if they were administered otherwise). For example, the two therapeutics may be administered at the same time or sequentially in any order at different points in time; however, if not administered at the same time, they should be administered sufficiently close in time so as to provide the desired therapeutic effect, preferably in a synergistic fashion. The combination cancer therapeutics can be administered separately, in any appropriate form and by any suitable route. When the components of the combination cancer therapeutics are not administered in the same pharmaceutical composition, it is understood that they can be administered in any order to a subject in need thereof. For example, a first therapeutically effective regimen can be administered prior to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of the second cancer therapeutic as per the invention, to a patient in need thereof.

Preferably the combined administration of an inhibitor of SUV39H1 with an immune checkpoint modulator according to the invention leads to a synergistic anti-cancer effect.

### Kit of parts preparations

The present application also encompasses preparations containing an inhibitor of SUV39H1 as previously described and at least one immune checkpoint modulator as also described above, as a combined preparation for simultaneous, separate or sequential use in cancer treatment. According to such preparations in the form of kit-of-parts" the individual active compounds (i.e., the inhibitor of SUV39H1 and the at least one immune checkpoint modulator), represent therapeutic agents and are physically separated, provided that the use of those compounds, either simultaneously, separately or sequentially, produces the new and unexpected joint therapeutic effect as herein described that is not attained by the compounds independently of each other. Indeed as demonstrated by the results below, the claimed combination of active ingredients did not represent a mere aggregate of known agents, but rather a new combination with the surprising, valuable property that the combined anti-tumor effect is much more important that the simple addition of the anti-tumor effects that are observed, when those active ingredients are used separately.

Both active ingredients may be thus formulated into separate compositions or into a unique composition.

The therapeutic agents as per the invention can be suitably formulated and introduced into a subject or the environment of the cell by any means recognized for such delivery.

Such compositions typically include the agent and a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration.

The invention will further be illustrated in view of the following example.

### BRIEF DESCRIPTION OF THE FIGURES

**Figure 1****:** Effect of anti-PD-1 treatment on tumor growth in Suv39h1-WT *vs.* Suv39h1-KO mice. Tumor growth curves in littermate WI mice with anti-PD-1 treatment (C) or not (A) and in Suv39h1 KO mice with anti-PD-1 treatment (D) or not (E).
**Figure 2****:** Cellular immune response in WT and Suv39h1 KO mice treated or not with an anti-PD-1 immunotherapy.

### EXAMPLE

### Materials and Methods

*Suv39h1* KO and littermate WT male mice were injected subcutaneously with 0.5x10⁶ B16-OVA melanoma cells into the lateral flank.

When tumors became palpable, usually within a week, mice where intraperitoneally injected with anti-PD1 (Bio X Cell, RMP-14) administrated at a dose of 7.5 mg/Kg body weight per dose twice/week. Cold PBS was injected to control groups.

Tumor growth was measured three times a week using a manual caliper.

Cellular immune response (i.e., Anti-OVA immune response) was tested using enzyme-Linked ImmunoSpot (ELISPOT) assay for IFN_{γ} in the blood of tumor bearing mice treated or not with anti-PD-1 immunotherapy 13 days after establishment of the tumor.

Elispots were performed after overnight *in vitro* re-stimulation with class I MHC OVA peptide (257-264).

### Results

### Anti-PD-1 treatment is more effective in Suv39h1-KO than Suv39h1-WT mice

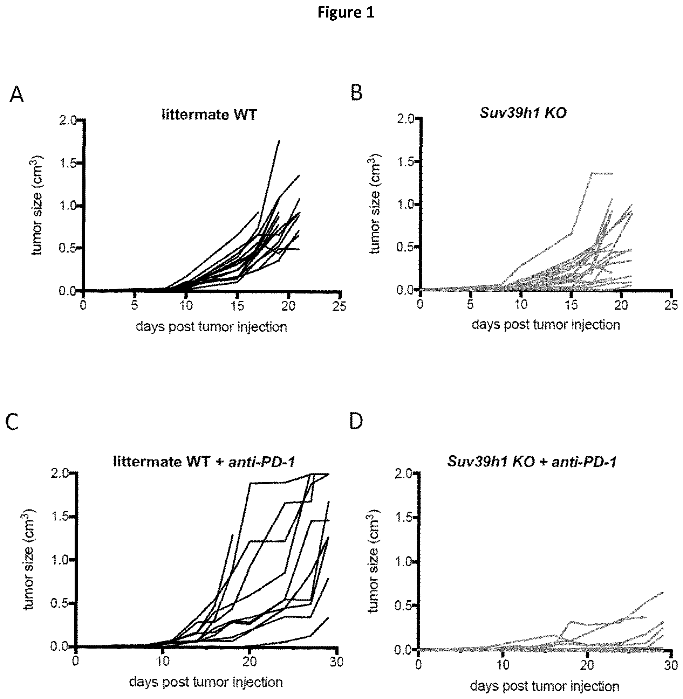

We observed that the syngeneic melanoma tumor cell line B16, grew slightly less in SUV39H1-knockout mice (KO) compared to SUV39H1-wild type (WT) littermates, indicating that the enzyme participates to the control of tumor development. Anti-PD-1 treatment in WT mice (figure 1, left panel) induces a slight delay in tumor growth. Interestingly, as observed in figure 1, administration of anti-PD-1 Ab to mice bearing palpable B16 tumors was impressively efficient in controlling tumor growth in *Suv39h1-*knockout mice (KO) compared to Suv39h1-wild type (WT) littermates. Indeed anti-PD1 treatment in WT mice does not induce tumor rejection, whereas in KO mice only 1/4 tumors growths and with a delayed kinetics.

This result highlights the synergy of combining an anti-PD1 blockade to a SUV39H1 blocking agent for the treatment of cancer.

### Anti-PD-1 treatment induces enhanced anti-tumor immune responses in Suv39h1 KO mice

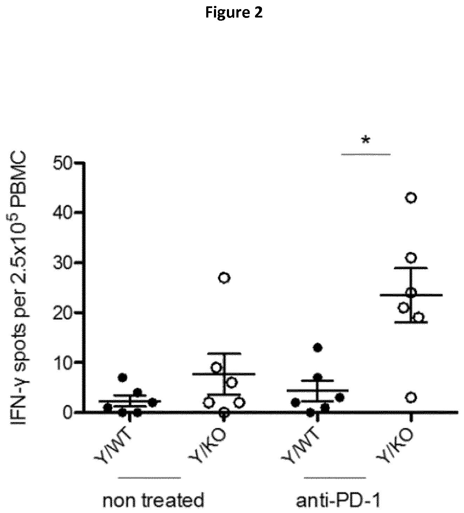

Anti-tumor immune responses in WT and *Suv39h1* KO mice were analyzed using OVA as a surrogate tumor antigen. IFNg-Elispots were tested in the blood of the mice bearing B16-OVA tumors (see figure 2).

We conclude that Suv39h1 defective mice mount more effective anti-tumor immune responses after immunotherapy using anti-PD-1.

## Claims

1. An inhibitor of H3K9 histone methyl transferase SUV39H1 gene expression selected from anti-sense oligonucleotide constructs, siRNAs, shRNAs and ribozymes targeting SUV39H1 mRNA, for use in combination with at least one inhibitor of an inhibitory immune checkpoint molecule that is an anti-Programmed Death-Ligand 1 (PD-L1) antibody, in the treatment of cancer.

2. The inhibitor of H3K9 histone methyl transferase SUV39H1 gene expression for use according to claim 1, which is for simultaneous, separate or sequential use in combination with the anti-PD-L1 antibody.

## Patentansprüche

1. Inhibitor der H3K9-Histon-Methyltransferase-SUV39H1-Genexpression, ausgewählt aus Antisense-Oligonukleotid-Konstrukten, siRNAs, shRNAs und Ribozymen, die auf SUV39H1-mRNA gerichtet sind, zur Verwendung in Kombination mit mindestens einem Inhibitor eines inhibitorischen Immun-Checkpoint-Moleküls, bei dem es sich um einen Anti-Programmed-Death-Ligand-1(PD-L1)-Antikörper handelt, bei der Behandlung von Krebs.

2. Inhibitor der H3K9-Histon-Methyltransferase-SUV39H1-Genexpression zur Verwendung nach Anspruch 1, welcher zur gleichzeitigen, getrennten oder aufeinanderfolgenden Verwendung in Kombination mit dem Anti-PD-L1-Antikörper bestimmt ist.

## Revendications

1. Inhibiteur de l'expression génique de l'histone méthyltransférase H3K9 SUV39H1 choisi parmi des constructions d'oligonucléotides antisens, des ARNsi, des ARNsh et des ribozymes ciblant l'ARNm de SUV39H1, pour une utilisation dans le traitement du cancer en combinaison avec au moins un inhibiteur d'une molécule de point de contrôle immunitaire inhibiteur qui est un anticorps anti-Programmed Death-Ligand 1 (PD-L1).

2. Inhibiteur de l'expression génique de l'histone méthyltransférase H3K9 SUV39H1 pour une utilisation selon la revendication 1, qui est destiné à une utilisation simultanée, séparée ou séquentielle en combinaison avec l'anticorps anti-PD-L1.
